# EUROPEAN PATENT SPECIFICATION

(11) **EP 1 968 653 B1**
(45) Date of publication and mention of the grant of the patent: **27.02.2013**
(21) Application number: 06788352.0
(22) Date of filing: 25.07.2006
(51) Int. Cl.: A61L 9/22, A61L 2/04, B01J 19/08, C01B 13/02, C01B 13/10

(54) **APPARATUS AND METHOD FOR SANITIZING AIR AND SPACES**
GERÄT UND VERFAHREN ZUR REINIGUNG VON LUFT UND RÄUMEN
DISPOSITIF ET PROCEDE PERMETTANT D'ASSAINIR AIR ET ESPACES

(30) Priority: 30.11.2005 US 289363
(43) Date of publication of application: 17.09.2008
(73) Proprietor: Airocare, Inc., Rockville, MD 20852 (US)
(72) Inventor: CHAMBERS, William, R., Vienna, VA 22182 (US); LIMA, Carlos, Antonio, Santiago, XX (CL); MCDONALD, Robert, D., Great Falls, VA 22026 (US); WOODBRIDGE, Terrance, O., Cary, NC 27519 (US)
(74) Representative: Hedley, Nicholas James Matthew
(86) International application number: PCT/US2006/028734
(87) International publication number: WO 2007/064368

(56) References cited:
- EP-A2- 1 125 588
- WO-A1-00/78670
- DE-A1- 1 923 081
- US-A- 2 778 443
- US-A1- 2003 121 770
- US-A1- 2005 186 108
- US-B1- 6 228 149

## Description

### FIELD OF THE INVENTION

The present invention relates to a method and apparatus for sanitizing air and spaces through the generation of reactive oxygen species.

### BACKGROUND OF THE INVENTION

DE1923081 discloses the ionisation of air and other free gases, whereby the gas is sucked through a ventilator into a cylindrical vessel containing an ultra-violet lamp or other radiation source. The emitted electrons are accelerated by an electric field between the vessel wall and a concentric screen. Ionisation is achieved by collision of the electrons with the gas molecules.

Temperature changes and changes in the moisture in the air feeding into heating, ventilation, and air-conditioning (HVAC) systems increases the number micro-organisms in the air, producing increased colonies of certain fungi and bacteria, both of which are potentially harmful.

HVAC systems in office buildings, as well as hospitals, can be a source of various pathogens which spread infectious micro-organisms from one zone to another - a principal cause of Sick Building Syndrome, recognized by the World Health Organization as a threat to healthy work and living environments.

The purification of environments can be achieved through the use of ozone. Ozone has been used to purify air conditioning systems in buildings and to sanitize warehouses where products are stored. Despite its widespread use, this basis technique has the disadvantage of accumulating more ozone than is necessary in the treated environment, requiring the elimination of the excess ozone. Several different improvements in this method have been made in an attempt to control the levels of ozone in the environment being treated.

One such improvement provides high initial levels of ozone to the environment sufficient to produce the desired bacteriostatic or bacteriocidal effect. Later the levels of ozone are reduced so that they do not produce harmful effects to the products being treated or to humans in the environment.

However, the majority of the known systems for purifying closed areas with ozone are based on an ozone generator that utilizes a source of concentrated oxygen, for example bottled oxygen or a known pressurized oxygen generating system utilizing static discharge. When ozone is generated from a source of concentrated oxygen, the level of oxygen in the enclosure may rise along with the level of ozone. The increase in oxygen levels is due to the breakdown of ozone partially into new molecules of oxygen. An increase in the level of oxygen in enclosures containing natural perishable products enhances cellular metabolism and thus is detrimental to the storage of the perishable products.

One known method is applied to substantially closed rooms or rooms with a controlled atmosphere. The substantially closed room includes a closed-circuit air conditioning system, such as a cooling system, for the preservation of perishable natural products. A known ozone generator is placed in proximity with the substantially closed room such that the ozone generator can draw in air from within the substantially closed room and liberate ozone into the substantially closed room. In contrast to other known ozonation systems, the known method utilizes oxygen from the air of the room in which the purification treatment is being applied to generate ozone. Because the method converts oxygen from the air into ozone, no increase in oxygen levels is observed in the closed room. Rather, the gaseous equilibrium is shifted so that there is maintenance of the level of oxygen in the enclosure.

The oxidative character of the ozone has a bacteriostatic and fungistatic effect in the short term, followed by a bacteriocidal and fungicidal effect. These effects combine with the lowered metabolism in a temperature cooled environment to reduce ripening, retard spoilage and thus preserve natural perishable products stored in the room.

However, the system does not provide an optimal means for efficiently sanitizing the air within the closed room.

### SUMMARY OF THE INVENTION

Therefore, it is an object of the present invention to provide an apparatus and method for generating reactive oxygen species and treating the air to be sanitized with the generated reactive oxygen species in order to efficiently sanitize air.

To attain the above object, according to a first aspect of the invention, there is provided an apparatus for sanitizing air according to claim 1.

Preferably, the reactive oxygen species generated include at least one of singlet oxygen, atomic oxygen, superoxide, hydrogen peroxide, hydroxyl radical, and peroxynitrite.

The reaction unit preferably further generates ozone from the oxygen in the air and the generated ozone is discharged with the air from the reaction unit, wherein the ozone in the sanitized air acts as a sanitizing agent for sanitizing surfaces.

The apparatus preferably further includes an intake port for the receiving air to be sanitized, and an exhaust port for discharging the substantially sanitized air, wherein the reaction unit is disposed between the intake port and the exhaust port.

Preferably, the apparatus further includes a power supply capable of a high frequency and high voltage output electrically coupled with the reaction unit to create a corona discharge which splits the oxygen in the air into reactive oxygen species.

A second aspect of the invention provides a method for sanitizing air according to claim 7.

These and other exemplary features and advantages of the present invention will become clear from the following description with reference to the accompanying drawings.

### BRIEF DESCRIPTION OF THE DRAWINGS

The foregoing and other exemplary purposes, aspects and advantages will be better understood from the following detailed description of an exemplary embodiment of the invention with reference to the drawings, in which:
FIG. 1 shows a block diagram of the apparatus of the exemplary embodiment of the invention.
FIG. 2 shows a perspective view of an exemplary embodiment of the reaction unit of the invention.
FIG. 3 shows an exploded perspective view of the reaction unit of FIG. 2.
FIG. 4 shows a further exploded perspective view of the reaction unit of FIG. 2.
FIG. 5 shows an exploded perspective view of the reaction chamber of FIG. 2.
FIG. 6 shows a perspective view of another embodiment of the reaction unit.

### DETAILED DESCRIPTION OF THE INVENTION

Referring now to the drawings, and more particularly to Figures 1-6, there are shown exemplary embodiments of the method and structures according to the present invention.

FIG. 1 shows an exemplary embodiment of the apparatus 10 for sanitizing air. The apparatus 10 includes an intake port 12 for receiving air to be sanitized and an exhaust port 14 for discharging substantially sanitized air. A reaction unit 16 is disposed between the intake port 12 and the exhaust port 14. The reaction unit 16 generates reactive oxygen species from oxygen (O₂) in the air received through the intake port 12.

The air received through the intake port 12 is preferably ambient air from the environment The introduction of air into the reaction unit 16 may be mediated through a forced suction or by natural suction. When mediated through a forced suction, the apparatus 10 may contain a turbine which draws air into the reaction unit 16 through the intake port 12. Preferably, the air is drawn through a filter to remove dust and other macroscopic impurities that may be present in the air to be sanitized before the air enters the reaction unit 16.

The reaction unit 16 splits the oxygen in the air into large amounts of reactive oxygen species. The reactive oxygen species generated may include singlet oxygen (lO₂), ozone (O₃), atomic oxygen (O), superoxide (O₂-), hydrogen peroxide (H₂O₂), hydroxyl radical (OH-), and peroxynitrite (ONOO-). Even though many reactive oxygen species have a short half-life, they are effective sanitizing agents. Thus, as the air passes through the reaction unit 16, a large percentage of the airborne contaminants in the air received through the intake port 12 are neutralized by the generated reactive oxygen species before the air is exhausted through the exhaust port 14. In this manner, the reactive oxygen species generated in the reaction unit 16 act as a sanitizer of the air passing through the reaction unit 16.

One of the reactive oxygen species generated by the reaction unit 16 is ozone (O₃). The generated ozone is introduced into the air in the reaction unit 16, and the ozone also acts as a sanitizer of the air and environment. The ozone generated in the reaction unit 16 may be discharged with the air through the exhaust port 14. The ozone in the discharged air provides the beneficial preservative effects and acts as a sanitizer for any surfaces in the environment into which the air is discharged. Other reactive oxygen species, such as hydrogen peroxide, may also be discharged with the sanitized air and have sanitizing effects similar to ozone.

The apparatus may include a power supply 18 capable of producing high frequency and high voltage output. The power supply 18 is electrically coupled with the reaction unit 16 to create a corona discharge which splits the oxygen in the air into large amounts of reactive oxygen species. The power supply 18 provides power to the reaction unit 16.

The power supply 18 preferably includes an onboard intelligence 24 which enables the power supply 18 to adjust to changing conditions within the reaction unit 16. In this manner, the levels of reactive oxygen species generated within the reaction unit 16 can be maintained at desired levels regardless of changing conditions within the reactor unit 16. For example, the onboard intelligence 24 of the power supply 18 can compensate for variables that may affect the output of the reaction unit 16, such as changes in moisture content of the air to be sanitized or dust buildup within the reactor unit 16.

Further, the onboard intelligence 24 may allow for the dialing up and down of the levels of reactive oxygen species generated by the reaction unit 16. Preferably, the amount of reactive oxygen species generated by the reaction unit 16 is adjustable while maintaining continuous power to the reaction unit 16. However, one skilled in the art will recognize that the desired levels of reactive oxygen species may also be obtained by turning the reaction unit 16 on and off periodically.

The apparatus 10 may further include a source of ultravioiet (UV) light source 26 for illuminating the sanitized air discharged from the reaction unit 16 with UV light By illuminating the discharged air with specific frequencies of UV light, it is possible to neutralize the ozone in the discharged sanitized air. In particular, UVB light having a frequency between about 280 nm and 290 nm will effectively neutralize the ozone. Preferably, the UV light source 26 emits UVB light having a frequency of 285 mm to achieve optimal neutralization of the ozone. In this manner, the UV light source 26 can turned on and off as necessary to regulate the ozone levels in the air ultimately discharged into the environment while maintaining high reactive oxygen species levels within the reaction unit 16 to permit continued sanitization of the air.

Thus, by placing the UV light source 26 downstream from the exhaust port 14, the air may continue to be sanitized by the reactive oxygen species generated in the reaction unit 16, while the ozone levels of the discharged air can be selectively controlled by using the UV light source 26 to neutralize the ozone in the discharged air. The apparatus 10 may further include an adjustable arm which can move the UV light source 26 so that it can be positioned for maximum effectiveness. The UV light source 26 may be configured using reflective surfaces in the form of a mirrored center array with concave light areas so that the UV light can be dispersed in a desired fashion, for example through the entire width of a duct, in order to maximize the ozone neutralization capability of the apparatus 10.

The apparatus 10 may further include other means for neutralizing the generated ozone. For example, the apparatus may include a heat source or carbon filtration means to neutralize the ozone in the discharged air. Additionally, the apparatus 10 may include means for neutralizing any other generated reactive oxygen species that may be discharged with the sanitized air from the reaction unit 16.

The apparatus 10 may further include a plurality of sensors and modules 28 located within the apparatus 10 and throughout the environment into which the sanitized air is discharged. The sensors and modules 28 are used to measure pertinent variables, such as ozone levels, humidity, airflow, and temperature of the air in and around the apparatus 10. A programmable logic circuit (PLC) 30 may be used to measure the performance of the apparatus 10 based on data feedback from the plurality of sensors and modules 28. The PLC 30 may store this information locally or report the information to a controller 32 which can be linked to the apparatus 10 and to a central monitor and monitoring system 34, such as a computer or other dedicated device.

In this manner, the PLC 30 may be used to monitor and control multiple functions of the apparatus 10 and facilitate data collection, retention, and reporting of performance (such as ozone output, etc.). The PLC 30 may also be used to monitor and control the power supply 16 through the onboard intelligence 24. Thus, the onboard intelligence 24 may use the feedback from the sensors and modules 28 to appropriately adjust the reaction unit 16 to provide the desired levels of reactive oxygen species.

The PLC 30 may be appropriately configured to make the information accessible to a remote computing device 38 over a network 36. The network 36 may include any known communications or networking means, for example, a Wide Area Network (WAN), Local Area Network (LAN), Internet, Bluetooth®, or any wireless connection. Thus, the PLC 30 may permit regulation and diagnosis of the apparatus 10 remotely by the computing device 38 over the networking means 36. It is to be understood that one or more of the onboard intelligence 24, PLC 30, controller 32, and monitoring system 34, or functions thereof, may be provided on a single appropriately configured computing device for monitoring and controlling the functions of the apparatus 10.

FIGS. 2-4 shows perspective and exploded views of an exemplary embodiment of the reaction unit 16 of the invention. The reaction unit 16 may consist of one or more reaction chambers 100 in which the reactive oxygen species are generated. The reaction chambers 100 may be arranged in an array within a housing 102. The housing 102 may consist of round polyvinyl chloride (PVC) pipe of appropriate size. However, it is understood that the housing may be of any desired shape or material. For example, the housing 102 may consist of the duct work of an HVAC system.

Preferably, the reaction chambers 100 are held in place within the array by a coupler arranged on both ends of the reaction chambers 100. The coupler may include a clamp 103 for securing the reaction chambers 100 in a desired location within the array. A center support rod 112 may be included in the array and appropriately secured by the clamp 103 to provide additional structural integrity to the array. The coupler may further include an electrically conductive contact 104, 105 cooperatively shaped with the clamp 103 and contacting each of the reaction chambers 100 within the array. The contact 104 may be integrally formed with the clamp 103 or mechanically attached to the clamp 103 by an adhesive or mechanical fasteners 111.

The coupler preferably cooperates with an inner surface of the housing 102 to secure the reaction chambers 100 within the housing 102. The array may be fixed within the housing 102 using contact studs 109. The electrically conductive contact studs 109 pass through the housing 102 and interact with the coupler so as to fixedly secure the clamp 103 in relation to the housing 102 and electrically connect with the contacts 104,105. In this manner, the necessary electrical connections between the reaction chamber 100 of the reaction unit 16 and the power supply 18 may be achieved through the contact studs 109. However, one of ordinary skill in the art will recognize that the necessary electrical connections may be achieved by multiple means.

As shown in FIG. 5, the reaction chamber 100 consists of a glass tube 106 lined with an inner stainless steel mesh 107 and wrapped in an outer stainless steel mesh 108. This configuration has been found to create a very effective corona that is able to generate a large amount of reactive oxygen species without using a static discharge and without producing material amounts of off gases, such as nitrous oxide. While a round configuration for the reaction chamber is shown, the reaction chambers for generating reactive oxygen species may include different configurations and materials. For example, the reaction chambers may be formed of a glass tube 106 wrapped in stainless steel mesh with a copper tube coated with gold inside the glass tube at specific gaps. The reaction chambers may also be formed using appropriately configured plates of glass, ceramic or other materials with metal mesh on opposite sides. The particular configuration may be chosen to comport with the desired application of the apparatus 10.

As shown in FIG. 6, the apparatus 10 may include a plurality of reaction units 16 fluidly linked in a serial manner. In this manner, the air to be sanitized may be passed through multiple reaction units 16 in order to maximize the exposure of air to the reactive oxygen species, therefore greatly increasing the effective sanitation of the air. While a U-shape is shown, it is understood that the reaction units 16 may be arranged in any manner depending on the space constraints of the desired application of the apparatus 10.

The reaction units 16 may be linked using an appropriate connector 101 that links the housings 102 of the reaction units 16. The reaction units 16 may be linked using a butt-plate. The butt-plate may include all the necessary electrical connections for the reaction units 16 to eliminate high-voltage wiring and avoid wiring problems. This also makes servicing the apparatus 10 more streamlined and efficient. The necessary electrical connections between the reaction units 16 may be achieved using military lock in rotation connectors connecting the butt-plate 24 and reaction units 16. Additionally, each reaction unit 16 may have its own power supply 18 in order to make the apparatus 10 highly scalable.

The apparatus 10 may be configured for general room sanitation applications where the apparatus 10, or components thereof, may be placed in the duct work of an HVAC system servicing the room to be sanitized. Alternatively, the apparatus 10 may be incorporated into the HVAC system of a facility to generally sanitize the air in the facility. Additionally, the apparatus 10 may be used to sanitize air to be introduced to a room from an outside source (make up air), as well as to treat exhaust air to remove smells and contaminants before releasing the air into the environment.

The apparatus 10 may be placed directly into a duct of an HVAC system so that some of the components are external to the duct in order to balance or reduce the weight of the apparatus 10 and create less stress on the duct work. For example, one or more reaction units 16 may be placed in the duct so that the air in the duct flows directly through the reaction unit 16 resulting in the generated reactive oxygen species sanitizing the air passing through, and the generated ozone cleaning the duct and being dispersed into the environment. As described above, a UV light source 26 may be placed downstream from the reaction units 16 in the duct to regulate the dispersion of ozone into the environment.

The level of ozone maintained in the environment into which the sanitized air containing ozone is dispersed, for example a room or building, may vary from as low as 0.02 PPM to higher levels depending on regulations and safe operating conditions based on human presence. One skilled in the art will realize that the optimum level will be determined based on the size, configuration, and contents of the room. Further, one skilled in the art will recognize that the levels of ozone maintained in the environment used by people may be limited by governmental regulation. For example, OSHA regulations stipulate that eight hours of exposure to 0.1 PPM ozone is acceptable and that fifteen minutes of exposure to 0.3 PPM ozone is acceptable. Use of higher concentrations may be dangerous. In the preferred embodiment, the level of ozone will be controlled and maintained; for example by the PLC 30, in accordance with governmental regulations. Higher levels of reactive oxygen species and ozone may be used during unoccupied periods for additional sanitation.

While the description refers to sanitizing air to be discharged into a room, space, or environment, it is to be understood that the invention can be applied to any defined environment. For example, an environment may be defined by solid surfaces or barriers, such as walls or product packaging, or defined by streams of forced gases, such as air screens or air curtains. Alternatively, the environment may be simply defined by the specific requirements of a desired application of the invention.

An exemplary application of the apparatus 10 would be for sanitizing sensitive areas of medical facilities, such as acute care areas and operating rooms. For example, the air circulation system of an operating room may include a network of ducts and vents that allow for the circulating of the air within the room without taking in air from outside the room. The apparatus 10, or elements thereof, may be placed in the duct work so that the air in the operating room may circulate through one or more reaction units 16. By including a UV light source 26, when the room is in use, the UV light source 26 may be turned on to prevent ozone from being dispersed in the room. But when the room is not occupied, the UV light source 26 may be turned off, allowing the generated ozone to circulate throughout the room and remove contaminants from surfaces inside the room. It is to be understood that the apparatus 10 may be employed in a wide variety of medical applications. For example, the sterilization of medical equipment storage cabinets and rooms, such as endoscope cabinets, and the sanitization of other rooms of medical facilities, such as waiting rooms, bathrooms, and food production areas.

In a similar manner, the apparatus 10 may be utilized in food processing environments to sanitize the air while food is being processed with workers present, provide the beneficial preservative effects of ozone while food is being stored (before and after processing), and sanitize the air and surfaces while the processing room is vacant. The apparatus 10 may also be configured into food processing equipment so that food is treated as it moves through the equipment, for example on a conveyor belt, automatic cutters and slicers and inspection areas. The product may be tumbled to promote uniform treatment. The apparatus 1 0 may also be configured to be placed in containers, trailers, and rail cars or as a component to a refrigeration system of such containers, trailers, and rail cars to sanitize the air therein while providing the beneficial preservative effects of ozone to any products stored therein.

Other exemplary applications of the apparatus include the provision or incorporation of the apparatus 10 into: grocery store display cases, such as deli counters and meat, fish and poultry display cases; floral display cases, both refrigerated and non-refrigerated; and HVAC systems of various public transportation means, such as cars, buses, trains, subways, or aircraft. The invention may be employed in pressurized environments, such as aircraft and positively or negatively pressurized rooms and structures. The apparatus 10 may also be incorporated into packing and production line equipment that blows air into bags as products are packed and sealed to sanitize the air blown into the bag and preserve the product therein, or into equipment that is integrated into a production line to sanitize the air and product before packaging.

As noted above, the apparatus 10 may also be incorporated into the HVAC system of public buildings in order to generally treat the air within the buildings. In this manner, the apparatus 10 may be used to sanitize the air and eliminate odors in the buildings. For example, office buildings, restaurants, malls, and the like would be particularly appropriate applications due to the large numbers of people that occupy the buildings and the need to sanitize the air in the buildings to provide a healthier, cleaner and more desirable environment for the occupants. The apparatus 10 may further be employed to sanitize air that is to be exhausted out of buildings in order to eliminate or reduce contaminants and odors emitted from the building into the surrounding environment.

In another exemplary application of the invention, the apparatus 10 may include sensors 28 for detecting potentially harmful agents in the environment. For example, the apparatus 10 may be incorporated into an HVAC system of a building and include appropriate sensors 28 for detecting noxious chemical or biological agents that may be unlawfully or accidentally released in or around the building. The apparatus 10 may be appropriately controlled to automatically operate in response to a positive detection of such agents by the sensor 28 in order to sanitize the air and protect the occupants of the building from the harmful agents.

In yet another application of the invention, the sanitized air discharged into the environment may be directed through a nozzle or jet to permit directional control of the sanitized air. In this manner, the sanitized air can be actively directed to a specific location or area requiring the sanitizing effect of the discharged air. Similarly, the invention may be incorporated into a means for creating air curtains or air doors. For example, an air curtain can be created to substantially enclose a specified space in order to contain and control any undesirable odors or emissions from contents within the created space, or, alternatively, sanitize or preserve the contents within the created space.

In a further exemplary application of the invention, the apparatus 10 may be incorporated into vacuum cleaner devices, for example stand-alone or centralized vacuum cleaners, wet-dry vacuums, and carpet cleaners, in order to sanitize air discharged from the cleaner. In this manner, any contaminants and odors inhaled by the cleaner would be sanitized and not discharged into the environment in which the cleaner was being utilized.

## Claims

1. An apparatus for sanitizing air comprising:
a reaction unit (16) configured to generate ozone and a reactive oxygen species, through use of a corona discharge, from oxygen in air received in the reaction unit to be sanitized, said reaction unit comprising at least one reaction chamber (100) in which the reactive oxygen species are generated, the reaction chamber (100) comprising a glass tube (106) wrapped in an outer stainless steel mesh (108)
wherein the apparatus is configured so that airborne contaminants in the received air are substantially neutralized by the generated reactive oxygen species before the air is discharged from the reaction unit,
**characterized in that**:
the glass tube (106) of the reaction chamber (100) is lined with an inner stainless steel mesh (107).

2. The apparatus of claim 1, further comprising:
an intake port (12) for the receiving air to be sanitized; and
an exhaust port (14) for discharging the substantially sanitized air,
wherein the reaction unit (14) is disposed between the intake port and the exhaust port.

3. The apparatus of claim 1, further comprising:
a power supply (18) capable of a high frequency and high voltage output electrically coupled with the reaction unit (16) to create a corona discharge which splits the oxygen in the air into reactive oxygen species.

4. The apparatus according to claim 1, wherein said reaction unit (16) is configured to sanitize unfiltered air with the reactive oxygen species.

5. The apparatus according to claim 1, wherein the reaction unit (16) is configured to discharge the ozone with hydrogen peroxide.

6. The apparatus according to claim 1, wherein the reaction unit (16) is configured to discharge the generated ozone directly into the air to be sanitized.

7. A method for sanitizing air by the use of an apparatus according to any of the preceding claims comprising:
generating reactive oxygen species from oxygen in ambient air, said reactive oxygen species comprising at least one of ozone singlet oxygen, atomic oxygen, superoxide, hydrogen peroxide, hydroxyl radical, and peroxynitrite, and
sanitizing a flow of ambient air using the generated reactive oxygen species.

## Patentansprüche

1. Vorrichtung zum Reinigen von Luft mit:
einer Reaktionseinheit (16), die konfiguriert ist, um unter Verwendung von Koronaentladung Ozon und eine reaktive Sauerstoffspezies aus Sauerstoff in zu reinigender Luft, die in der Reaktionseinheit aufgenommen wird, zu erzeugen,
wobei die Reaktionseinheit wenigstens eine Reaktionskammer (100) aufweist, in der die reaktiven Sauerstoffspezies erzeugt werden, wobei die Reaktionskammer (100) ein Glasrohr (106) aufweist, das in ein äußeres Edelstahlgeflecht (108) eingehüllt ist,
wobei die Vorrichtung so konfiguriert ist, dass in der aufgenommenen Luft enthaltene Luftverunreinigungen im Wesentlichen durch die erzeugte reaktive Sauerstoffspezies neutralisiert werden, bevor die Luft aus der Reaktionseinheit abgeleitet wird,
**dadurch gekennzeichnet, dass**
das Glasrohr (106) der Reaktionskammer (100) mit einem inneren Edelstahlgeflecht (107) ausgekleidet ist.

2. Vorrichtung nach Anspruch 1, die des Weiteren aufweist:
eine Einlassöffnung (12) zum Aufnehmen der zu reinigenden Luft; und
eine Auslassöffnung (14) zum Ableiten der im Wesentlichen gereinigten Luft,
wobei die Reaktionseinheit (14) zwischen der Einlassöffnung und der Auslassöffnung angeordnet ist.

3. Vorrichtung nach Anspruch 1, die des Weiteren aufweist:
eine Energieversorgung (18), die zu hoher Frequenz und hohem Spannungsausgang fähig und mit der Reaktionseinheit (16) elektrisch verbunden ist, um eine Koronaentladung zu erzeugen, die den Sauerstoff in der Luft in reaktive Sauerstoffspezies spaltet.

4. Vorrichtung nach Anspruch 1, wobei die Reaktionseinheit (16) konfiguriert ist, um ungefilterte Luft mit der reaktiven Sauerstoffspezies zu reinigen.

5. Vorrichtung nach Anspruch 1, wobei die Reaktionseinheit (16) konfiguriert ist, um das Ozon mit Wasserstoffperoxid abzuleiten.

6. Vorrichtung nach Anspruch 1, wobei die Reaktionseinheit (16) konfiguriert ist, um das erzeugte Ozon direkt in die zu reinigende Luft abzuleiten.

7. Verfahren zum Reinigen von Luft durch die Verwendung einer Vorrichtung gemäß einem der vorhergehenden Ansprüche, wobei das Verfahren umfasst:
Erzeugen reaktiver Sauerstoffspezies aus Sauerstoff in Umgebungsluft, wobei die reaktiven Sauerstoffspezies wenigstens eines aufweisen von Ozon, Singulett-Sauerstoff, atomarem Sauerstoff, Superoxid, Wasserstoffperoxid, Hydroxyl-Radikal und Peroxynitrit, und
Reinigen eines Flusses von Umgebungsluft mittels der erzeugten reaktiven Sauerstoffspezies.

## Revendications

1. Dispositif permettant d'assainir l'air comprenant :
une unité de réaction (16) configurée pour générer de l'ozone et des dérivés réactifs de l'oxygène, en utilisant une décharge corona, à partir de l'oxygène dans l'air reçu dans l'unité de réaction à assainir, ladite unité de réaction comprenant au moins une chambre de réaction (100), dans laquelle les dérivés réactifs de l'oxygène sont générés, la chambre de réaction (100) comprenant un tube de verre (106) enveloppé dans une maille en acier inoxydable extérieure (108)
dans lequel le dispositif est configuré de sorte que les contaminants en suspension dans l'air reçu soient sensiblement neutralisés par les dérivés réactifs de l'oxygène générés avant que l'air ne soit évacué de l'unité de réaction,
**caractérisé en ce que** :
le tube de verre (106) de la chambre de réaction (100) est revêtu d'une maille en acier inoxydable intérieure (107).

2. Dispositif selon la revendication 1, comprenant en outre :
un orifice d'entrée (12) pour la réception de l'air à assainir ; et
un orifice d'évacuation (14) pour l'évacuation de l'air sensiblement assaini,
dans lequel l'unité de réaction (14) est disposée entre l'orifice d'entrée et l'orifice d'évacuation.

3. Dispositif selon la revendication 1, comprenant en outre :
une alimentation électrique (18) capable d'une sortie haute fréquence et haute tension électriquement couplée à l'unité de réaction (16) pour créer une décharge corona qui divise l'oxygène de l'air en dérivés réactifs de l'oxygène.

4. Dispositif selon la revendication 1, dans lequel ladite unité de réaction (16) est configurée pour assainir de l'air non filtré avec les dérivés réactifs de l'oxygène.

5. Dispositif selon la revendication 1, dans lequel l'unité de réaction (16) est configurée pour évacuer l'ozone avec du peroxyde d'hydrogène.

6. Dispositif selon la revendication 1, dans lequel l'unité de réaction (16) est configurée pour évacuer l'ozone généré directement dans l'air à assainir.

7. Procédé permettant d'assainir l'air en utilisant le dispositif selon l'une quelconque des revendications précédentes, comprenant les étapes suivantes :
générer des dérivés réactifs de l'oxygène à partir de l'oxygène contenu dans l'air ambiant, lesdits dérivés réactifs de l'oxygène comprenant au moins un gaz parmi l'ozone, l'oxygène singulet, l'oxygène atomique, le peroxyde, le peroxyde d'hydrogène, le radical hydroxyle et le peroxynitrite, et
assainir un flux d'air ambiant en utilisant les dérivés réactifs de l'oxygène générés.
